# EUROPEAN PATENT SPECIFICATION

(11) **EP 1 423 078 B1**
(45) Date of publication and mention of the grant of the patent: **09.11.2005**
(21) Application number: 02760957.7
(22) Date of filing: 20.08.2002
(51) Int. Cl.: A61F 13/64

(54) **ABSORBENT ARTICLE AND BELT ARRANGED TO BE ATTACHED TO AN ABSORBENT ARTICLE**
SAUGFÄHIGER ARTIKEL UND GÜRTEL ZUR BEFESTIGUNG AN EINEM SAUGFÄHIGEN ARTIKEL
ARTICLE ABSORBANT, ET CEINTURE DESTINEE A ETRE FIXEE A CET ARTICLE

(30) Priority: 30.08.2001 SE 0110289
(43) Date of publication of application: 02.06.2004
(73) Proprietor: SCA Hygiene Products, 405 03 Göteborg (SE)
(72) Inventor: FERNFORS, Ingemar, S-431 38 Mölndal (SE)
(74) Representative: Egeröd, Lisbeth
(86) International application number: PCT/SE2002/001475
(87) International publication number: WO 2003/017903

(56) References cited:
- US-A- 4 776 911
- US-A- 5 135 522
- US-A- 5 706 524

## Description

### Technical field

The present invention relates to an absorbent article such as a diaper and an incontinence guard comprising a liquid permeable topsheet, a liquid impervious backsheet and an absorbent body enclosed therebetween, whereby the article exhibits a front portion, a rear portion and a crotch portion arranged therebetween, and further exhibits belt portions being attached to the rear portion alternatively the front portion of the article and by means of first fastening means is arranged to be attached around the waist of the user and where said front portion alternatively the rear portion exhibits second fastening means intended to be attached against the belt portions in such a way that the article assumes a pantlike shape where the belt portions form a part of the waist portions of the pant.

### Background of the invention

Diapers and incontinence guards for incontinent adults usually exhibits a garment portion holding an absorbent body in place against the users body and fastening means, which hold the garment portion in place also when the user is moving. A common type of fastening means are adhesive tapes or hook and loop fasteners of the touch and close type, which directly attach the front and rear portions of the absorbent article to each other. It is further known, through e.g., EP-A-0 287 388, EP-A-0 409 307, EP-A-0 528 282, EP-A-0 605 012 and FR-A-2 586 558, to attach the front and rear portions of the article by means of a belt, at which the possibilities to adjust the fit are improved. On a common type of belt diaper, the belt portions are first attached around the waist of the patient and then the front portion of the diaper is attached to the outside of the belt using fastening means being arranged on the front portion of the article, whereby the outside of the belt functions as a reception surface for said fastening means.

Generally, a conventional diaper for children is applied having the child in a lying position. The attachment means are usually arranged on the rear portion and are attached to the front portion. This kind of application often requires the aid from another person. However, for an adult user it is more desirable to self be able to apply the incontinence guard. On a common type of belt diaper, the belt portions are therefore first attached around the waist. When the incontinence guard is fixed around the waist in this way, the user may reach after the rest of the incontinence guard between the legs and then the crotch portion of the incontinence guard is applied in the correct position by fastening the front portion of the diaper to the outside of the belt portions using hook and loop fasteners or tape tabs being arranged on the front portion and/or the belt portion. This design makes also possible for nursing personnel to apply the diaper on a standing person or for the user to apply the diaper on himself/herself in a standing position.

One problem is that the belt does not adapt itself after the body it is being applied on, causing discomfort for the wearer during use. The forces in the belt are being concentrated to the middle of the belt and arise from the fastening means arranged on the belt. Additionally, the forces are unequally distributed in the upper or lower part of the belt, respectively. The lower part is usually to tight and need to be able to give way and the upper part of the belt usually need to be closer to the body.

One way of solving this problem would be to use some kind of elastic means in order to make the product more flexible. However, the costs for the materials used in this kind of products must be kept low, since these products are mainly intended for one use only. To design the whole waistband in elastic material would therefore be costly. In addition, it is also difficult to combine a well working fastening system together with elastic materials in the belt. In SE 0004760-5, the belt portions are attached to the rear portion of the article via elastic side panels, improving the adaptation of the belt of the article around the waist of the wearer.

Another suggested solution is disclosed in SE 0003331-6, where the belt portions are being cut in a way to provide a bended belt and making it fit better on the wearer. However, this solution requires that adjustments must be made in the process for the manufacture of these articles.

Therefore, there exists a need for an absorbent product provided with a belt, wherein the belt adapts itself after the user wearing the diaper and which also feels comfortable to wear.

### Summary of the invention

The object of the present invention is to provide a belted diaper or incontinence guard being comfortable to wear and which fits persons of different sizes. This object is being solved in that the belt portions comprise a plurality of belt elements, whose main extension are in the transversal direction of the belt and which are mutually connected by means of threads or band, being essentially arranged in the longitudinal direction of the belt.

This design makes the belt adapt itself to the body shape of the wearer in that the belt elements aim to adjust themselves so that the belt takes the shortest way around said body. This distributes the forces equally over the belt, which leads to an increased comfort.

### Brief description of the drawings

In the following, the invention will now be closer described with reference to the embodiments shown on the enclosed drawings.
Fig. 1 schematically shows a perspective view from above of a diaper or incontinence guard according to the invention.
Figs. 2a-d show a view from above over different embodiments regarding a belt according to the invention.
Fig. 3 shows a view from above of a belt according to the invention provided with an edging.
Figs. 4a-b show a cut through the belt along the line IV-IV in Fig 3.
Fig. 5 shows a view from above of another embodiment according to the invention.
Fig. 6 shows a view from above of another embodiment according to the invention.
Fig. 7 shows a view from above of another embodiment according to the invention.

### Detailed description of the invention

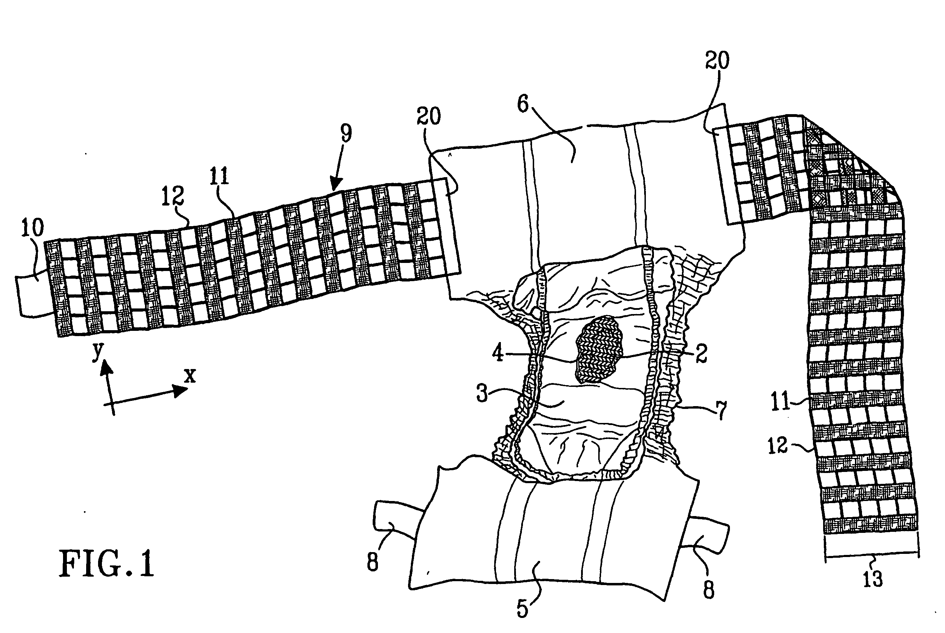

Fig. 1 shows an embodiment of a diaper or incontinence guard 1 comprising a liquid impermeable backsheet 2, a liquid permeable topsheet 3 and an absorbent body 4 enclosed therebetween. The liquid permeable topsheet 3 can consist of a nonwoven material, e.g., a spunbond material of continuous filaments, a meltblown material, a bonded carded fibrous web, a foam material or a perforated plastic film. The liquid impermeable backsheet 2 may consist of a plastic film, a nonwoven material coated with a liquid impervious material or a hydrophobic nonwoven material, which resists liquid penetration.

The backsheet material 2 and the topsheet 3 have a somewhat greater extension in the plane than the absorbent body 4 and extend outside the edges thereof. The layers 2 and 3 are connected to each other within the projecting portions thereof, e.g., by gluing or welding by heat or ultrasonic.

The absorbent body 4 can be of any conventional kind. Examples of commonly occurring absorbent materials are cellulose fluff pulp, tissue layers, highly absorbent polymers (so called superabsorbents), absorbent foam materials, absorbent nonwoven materials or the like. It is common to combine cellulose fluff pulp with superabsorbents in an absorbent body. It is also common to have absorbent bodies comprising layers of different material with different properties with respect to liquid acquisition capacity, liquid distribution capacity and storage capacity. It is well known to the person skilled in the art and does therefore not have to be described in detail. The thin absorbent bodies, which are common in for example baby diapers and incontinence guards often, comprise a compressed mixed or layered structure of cellulose fluff pulp and superabsorbent.

The diaper/incontinence guard is intended to enclose the lower part of the wearer's trunk like a pair of absorbent pants. It comprises a front portion 5, intended during use to be worn on the front part of the user's body, a rear portion 6, intended during use to be worn on the rear part of the user's body, and a crotch portion 7 located between the front and rear portions and which is intended to be worn in the crotch part of the user between the legs.

A pair of belt portions 9 is with one end attached, e.g., glued or ultrasonically welded, to the rear portion 6 of the diaper. The belt portions 9 are with their opposite ends intended to be fastened together by means of first fastening means 10. The fastening means 10 can be a hook-and-loop type fastener or tape tabs. The outside portions of the belt portions 9 comprise reception surface for the fastening means 10. The belt portions 9 exhibit on the inside, which is intended to be faced against the body, a soft, skin friendly material, preferably a nonwoven material. In one embodiment, the location of the fastening means 10 is on the right belt portion 9. It may naturally be the other way around, i.e., that the left belt portion carries the fastening means 10. However, since most people are right handed, are the embodiment described above probably the most preferred.

The definition hook and loop type fastener denotes a mechanical fastening device, where one part is a surface comprising a hook material and one part is a reception surface having a loop material. The loop material preferably consists of a nonwoven material. The term "hook material" shall not be regarded as being limited to a material having hooks, but comprises all materials having different shapes that are capable of mechanical attachment to complementary reception surfaces. The term "loop material" shall not be regarded as being limited to a material having separately formed loops and having the capability to attach to the hook members of a complementary hook material, but also comprise fibrous nonwoven material, in which the hook members from the complementary hook material surface can attach to the separate fibres without these fibres being shaped into separate loops.

The fastening means 8 of the front portion 5 is intended to be attached against the outsides of the belt portions 9, in order to fasten together the diaper/incontinence guard to the desired pantlike shape. The fastening means 8 can be hook and loop type fasteners or tape tabs. According to an altemative embodiment, the belt portions 9 are attached to the front portion 5 of the diaper and thus are intended to be fastened together on the back of the wearer. The fastening means 8 are then arranged on the rear portion 6 of the diaper.

The belt portions 9 are preferably a laminate of a carrier material, which forms the outside of the belt, and a soft nonwoven material, which forms the inside of the belt, which is intended to be in direct contact with the skin of the user. A suitable nonwoven material for the inside of the belt can be a spunbond material of e.g. polypropylene- or polyethylene fibres. Conjugate fibres may also be used. Another suitable nonwoven material can be a carded thermobonded material of e.g., polypropylene-, polyester- or conjugate fibres. As carrier material can for instance nonwoven or another suitable material be used. The carrier material should be adapted to function as a reception surface for both the attachment means 8 and 10. Also elastic laminates are suitable to use as material in the belt portions. The width of the belt portions 9 should be between 5-20 cm, preferably between 7-15 cm. Additionally, the belt portions can have a variable length 13, for example be wider at the attachment 20 to the rear portion 6 of the article and then be tapering along the longitudinal direction x towards the end portions of the belt portions.

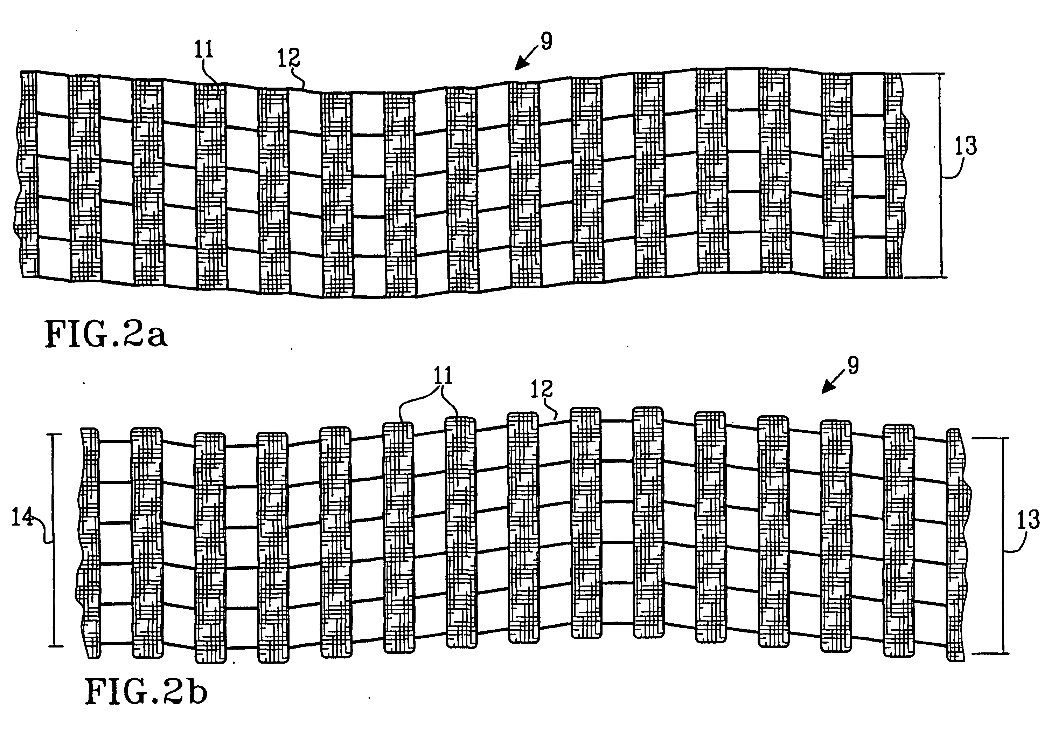

According the present invention the belt portions 9 comprise a plurality of belt elements 11, whose main extension are in the transversal direction y of the belt, i.e., the direction being perpendicular against the longitudinal direction x of the belt. Said belt elements 11 are mutually connected to each other by means of threads or bands extending along the belt, being arranged in the longitudinal direction x of the belt (see Fig. 2a). This design leads to that each belt element 11 can move upwards or downwards i.e., in the transversal direction y of the belt in respect of the adjacent belt elements 11. The belt adapts itself to the body shape of the wearer in that the belt elements aim to adjust themselves so that the belt takes the shortest way around said body. This distributes the forces equally over the belt, leading to an increased comfort. The belt will also allow a certain breathablity since there is a gap between the belt elements 11, making it possible for air to pass, which further contributes to the increased comfort during usage of the belt. It is preferred that said threads/bands 12 are attached on the outside of the belt elements 11 or alternatively through said belt elements 11 so that said threads/bands 12 not lie close to the skin of the wearer during use of the belt.

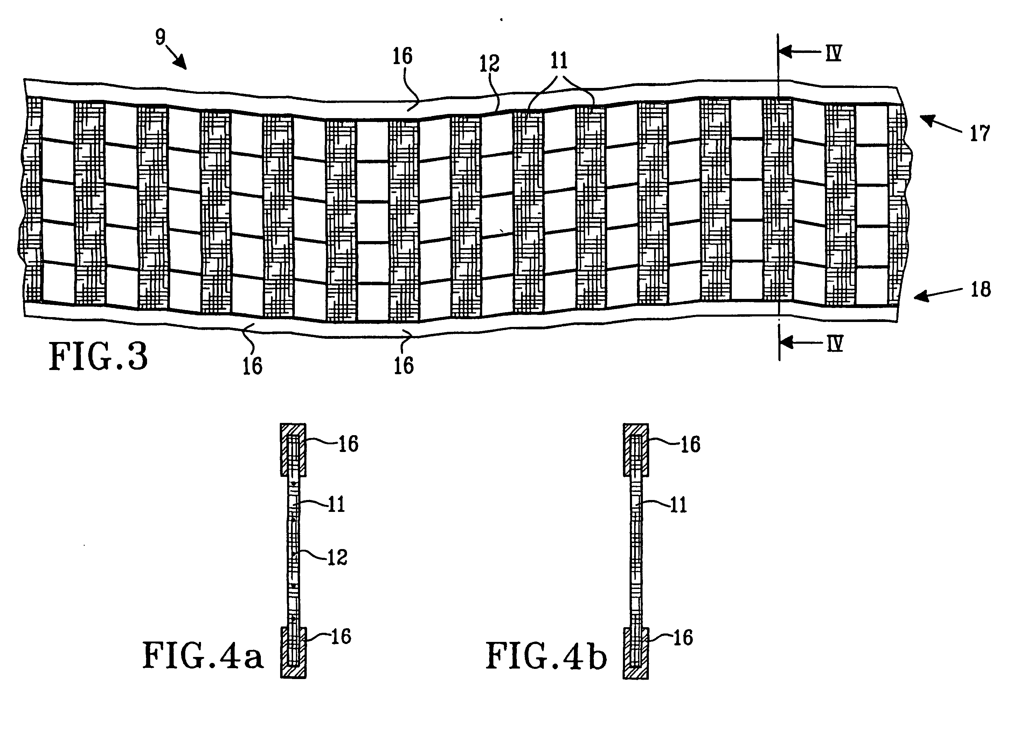

The threads or band 12 preferably extend continuously along the whole length of the belt portions 9 along the longitudinal direction x of the belt according to Fig. 4b (being a cut through one belt element 11 in Fig. 3), where it is visible that the threads/bands 12 extend without interruption along the belt portion 9 and each element is attached on their positions along the threads/bands 12. The material used in the threads/band 12 are preferably slightly flexible and can be extended to a certain length but not flex back completely to the original length after being extended, in order to allow a even better adaptation of the belt to the body of the user. The threads/bands 12 can mutually have different characteristics in the transversal direction y regarding elasticity and flexibility. It is also possible to use non-elastic materials in the threads/bands 12. Alternatively, the threads/bands 12 may extend discontinuously along the longitudinal direction x, i.e., only separately connect two belt elements 11 according to Fig. 4a (being a cut through a belt element 11 in Fig. 3), where it is visible that the threads/band 12 extend discontinuously along the belt portion 9). Then, two adjacent belt elements 11 are connected using discrete threads/band 12, which do not continue further to the next belt element 11. The threads/bands 12 can mutually in the transversal direction y have different characteristics regarding elasticity and flexibility. The material in the belt portions must be stiff enough so that it keeps its width 14 between the threads/bands 12 in the state before use. Each individual band 12 must not either cause too high transverse forces. If the material used in the belt elements 11 is stiff, more threads/bands 12 are generally required. The number of threads/bands 12 according to the present invention must be more than one, but the choice of materials for said threads/bands 12 and the corresponding belt element thus estimates the optimal number of threads/bands 12. If the belt portions 9 only are designed with bands 12, then these can be arranged next to each other and still function as the belt according to the present invention. The belt elements can then still move in y direction and thus result in an adaptation after the body of the user.

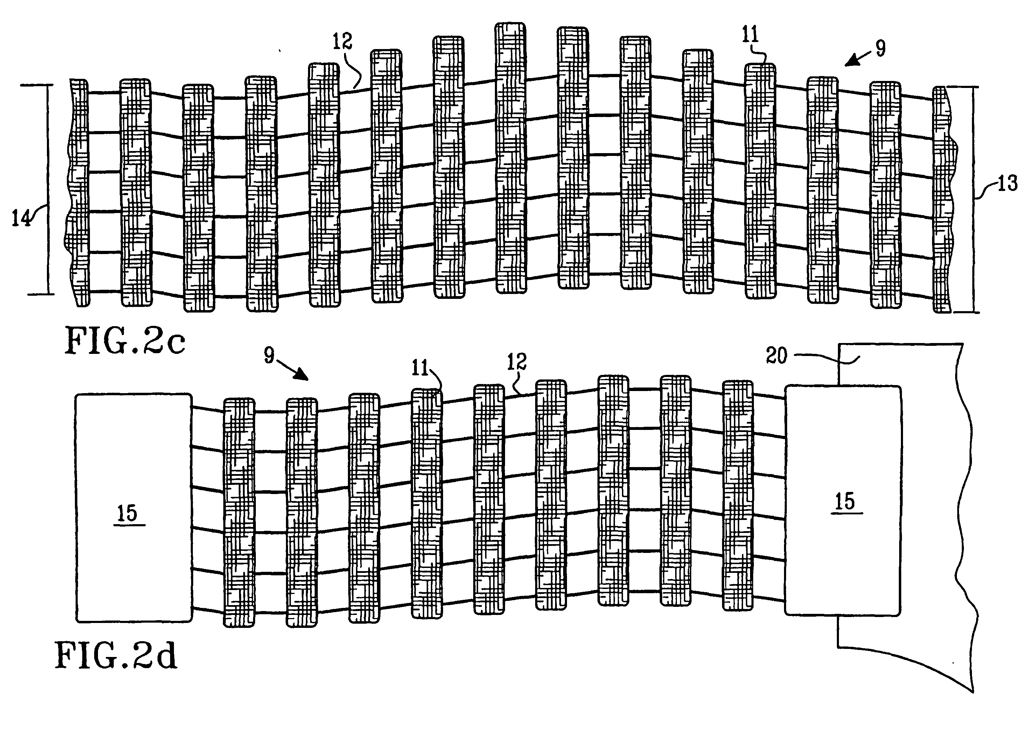

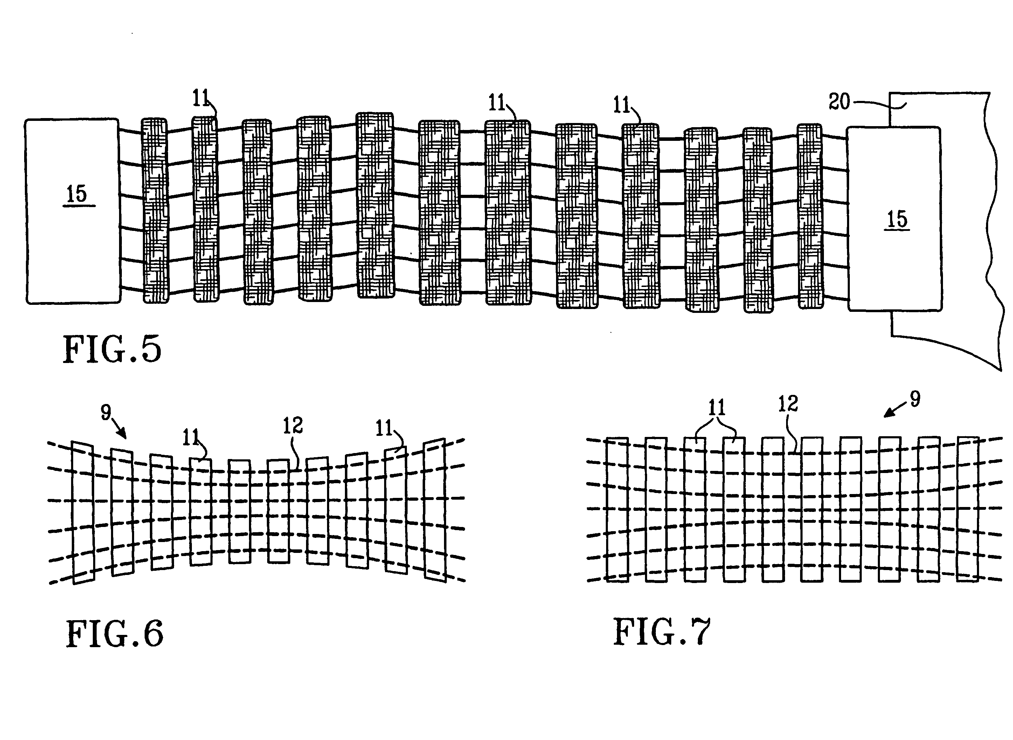

The length 13 of the belt elements 11 can vary between 0.3 cm and 20 cm, preferably between 0.5 and 2 cm (see Fig. 2a). Each individual element 11 can have a variable width 19, (see Fig. 5). The elements 11 as shown in the drawings have an essentially rectangular shape. However, the belt elements 11 according to the present invention may also exhibit other shapes, such as rounded or oval shapes. The length of the individual belt elements 11 in the transversal direction y is here denoted 13 and is in one embodiment substantially equally long as the distance 14 between the outermost threads/bands 12 between the belt elements 11. In another embodiment, the distance 14 can be less than the length 13 of the belt elements 11, i.e., the belt elements 11 protrude a bit along the longitudinal direction x of the belt portions 9 (see Fig. 2b). In this case, the belt elements 11 protrude about 5-15 mm from the outer threads/bands of belt portions 9. The belt elements 11 can protrude from the upper edge portion 17 of the belt portions 9 and/or from the lower edge portion 18 of the belt portions 9. The belt elements can, as shown in Fig. 2c, exhibit a variable length 13 along the longitudinal direction x of the belt portions.

The belt portions 9 can also comprise portions of belt elements 11 connected with threads/band 12 according to the description above and portions exhibiting belt material 15 without threads (see Fig. 2d). The belt portions can for example be designed with wider belt elements with or without threads/band 12 at the attachment 20 to the rear portion 6 or at the fastening means 10 or at another location along the belt portions 9.

The upper edge portion 17 of the belt portions 9 and/or the lower edge portion 18 can entirely or partly exhibit an edging 16 along the longitudinal direction x of the belt portions (see Fig. 3). The edging 6 can either only be arranged along one side of the belt portions 9 or being folded over the upper edge portion 17 and the lower edge portion 18 of the belt portions 9 (see Fig. 4a-b). The edging 16 can also provide an increased comfort for the wearer of an article according to the invention. The edging 16 can also assist in limiting the movements in the y direction of the belt elements 11.

The threads/bands 12 can also be arranged exhibiting a variable outer distance 14 between the threads/bands 12 (see Fig. 6 and 7) in the longitudinal direction x of the belt portions. This may yield belt portions 9 having belt elements 11, exhibiting a variable length 13 in the transversal direction y of the belt (see Fig. 6), but the belt elements 11 can also maintain the same length 13 along the transversal direction y of the belt (see Fig. 7). These designs can be advantageous for the purpose to adapt the belt on a user

The belt according to the present invention can, as have been described in the above-mentioned embodiments, be varied in endless variations depending on the user's needs. The variations of the body shape and size for children and adults can be enormous and each age group and size group will define an optimal design and choice of material for each group. Thus, the invention is intended to comprise belt portions for incontinence guards as well as diapers for all ages.

The invention is of course not limited to the above described and on the drawing showed embodiment but can be modified within the scope of the appended claims.

## Claims

1. Absorbent article such as a diaper and an incontinence guard comprising a liquid permeable topsheet (3), a liquid impervious backsheet (2) and an absorbent body (4) enclosed therebetween, whereby the article exhibits a front portion (5), a rear portion (6) and a crotch portion (7) arranged therebetween, and further exhibits belt portions (9), being attached to the rear portion (6) alternatively the front portion (5) of the article and which are intended by means of first fastening means (10) to be attached around the waist of the user and where said front portion (5) alternatively the rear portion (6) exhibits second fastening means (8) intended to be attached against the belt portions (9) in such a way that the article assumes a pantlike shape where the belt portions (9) form a part of the waist portions of the pant,
**characterised in,**
**that** said belt portions (9) comprise a plurality of belt elements (11) whose main extension are in a transversal direction (y) of the belt and which are mutually connected by means of threads or bands (12), being arranged in the longitudinal direction (x) of the belt.

2. Absorbent article according to claim 1,
**characterised in,**
**that** the threads/bands (12) extend discontinuously along the belt portions (9).

3. Absorbent article according to any of the claims 1 or 2,
**characterised in,**
**that** said threads or bands (12) are elastic.

4. Absorbent article according to any of the claims 1 or 2,
**characterised in,**
**that** said threads or bands (12) are non-elastic.

5. Absorbent article according to any of the claims 1 or 2,
**characterised in,**
**that** said threads or bands (12) partly comprise non-elastic threads/band and partly comprise elastic threads/band.

6. Absorbent article according to any of the preceding claims,
**characterised in,**
**that** an upper edge portion (17) of the belt portions (9) and/or a lower edge portion (18) of the belt portions (9) entirely or partly exhibit an edging (16).

7. Absorbent article according to any of the preceding claims,
**characterised in,**
**that** the belt elements (11) protrude in the transversal direction (y) direction of the belt from outer threads/band (12) of the belt portions (9).

8. Absorbent article according to any of the preceding claims,
**characterised in,**
**that** the belt elements (11) exhibit different lengths (13) in the transversal direction (y) of the belt along the longitudinal direction (x) of the belt.

9. Absorbent article according to any of the preceding claims,
**characterised in,**
**that** the belt elements (11) exhibit different widths (19) along the longitudinal direction (x) of the belt.

10. Absorbent article according to any of the preceding claims,
**characterised in,**
**that** said threads/band (12) are attached on the outside of the belt elements (11) alternatively through said belt elements (11), so that said threads/bands (12) do not lie close to the skin of the user during use of said belt.

## Patentansprüche

1. Absorbierender Gegenstand wie beispielsweise eine Windel und ein Inontinenzschutz, umfassend eine flüssigkeitsdurchlässige Oberlage (3), eine flüssigkeitsundurchlässige Decklage (2) und einen Absorptionskörper (4), der dazwischen eingeschlossen ist, wobei der Gegenstand einen Vorderabschnitt (5), einen Hinterabschnitt (6) und einen Schrittabschnitt (7), der dazwischen angeordnet ist, aufweist, und ferner Gürtelabschnitte (9) aufweist, die an dem Hinterabschnitt (6) bzw. alternativ dem Vorderabschnitt (5) des Gegenstandes angebracht sind und die dazu gedacht sind, mittels einer ersten Befestigungseinrichtung (10) um die Taille des Verwenders angebracht zu werden und wobei der Vorderabschnitt (5) bzw. alternativ der Hinterabschnitt (6) eine zweite Befestigungseinrichtung (8) aufweist, die dazu gedacht ist, derart gegen die Gürtelabschnitte (9) angebracht zu werden, dass der Gegenstand eine höschenähnliche Form annimmt, wobei die Gürtelabschnitte (9) einen Teil der Taillenabschnitte des Höschens bilden,
**dadurch gekennzeichnet, dass**
die Gürtelabschnitte (9) mehrere Gürtelelemente (11) umfassen, deren Haupterstreckung in einer Querrichtung (y) des Gürtels verläuft und die mittels Fäden oder Bänder (12), die in der Längsrichtung (x) des Gürtels angeordnet sind, miteinander verbunden sind.

2. Absorbierender Gegenstand nach Anspruch 1, **dadurch gekennzeichnet, dass** sich die Fäden/Bänder (12) entlang der Gürtelabschnitte (9) diskontinuierlich erstrecken.

3. Absorbierender Gegenstand nach einem der Ansprüche 1 oder 2, **dadurch gekennzeichnet, dass** die Fäden oder Bänder (12) elastisch sind.

4. Absorbierender Gegenstand nach einem der Ansprüche 1 oder 2, **dadurch gekennzeichnet, dass** die Fäden oder Bänder (12) nicht elastisch sind.

5. Absorbierender Gegenstand nach einem der Ansprüche 1 oder 2, **dadurch gekennzeichnet, dass** die Fäden oder Bänder (12) teilweise nicht elastische Fäden/Bänder und teilweise elastische Fäden/Bänder umfassen.

6. Absorbierender Gegenstand nach einem der vorstehenden Ansprüche, **dadurch gekennzeichnet, dass** ein oberer Kantenabschnitt (17) der Gürtelabschnitte (9) und/oder ein unterer Kantenabschnitt (18) der Gürtelabschnitte (9) vollständig oder teilweise eine Borte (16) umfasst.

7. Absorbierender Gegenstand nach einem der vorstehenden Ansprüche, **dadurch gekennzeichnet, dass** die Gürtelelemente (11) in der Querrichtung (y) des Gürtels von den äußeren Fäden/Bändern (12) der Gürtelabschnitte (9) vorragen.

8. Absorbierender Gegenstand nach einem der vorstehenden Ansprüche, **dadurch gekennzeichnet, dass** die Gürtelelemente (11) entlang der Längsrichtung (x) des Gürtels unterschiedliche Längen (13) in der Querrichtung (y) des Gürtels aufweisen.

9. Absorbierender Gegenstand nach einem der vorstehenden Ansprüche, **dadurch gekennzeichnet, dass** die Gürtelelemente (11) entlang der Längsrichtung (x) des Gürtels unterschiedliche Breiten (19) aufweisen.

10. Absorbierender Gegenstand nach einem der vorstehenden Ansprüche, **dadurch gekennzeichnet, dass** die Fäden/Bänder (12) an der Außenseite der Gürtelelemente (11) bzw. alternativ durch die Gürtelelemente (11) angebracht sind, so dass die Fäden/Bänder (12) während dem Gebrauch des Gürtels nicht nahe der Haut des Verwenders liegen.

## Revendications

1. Article absorbant, tel qu'une couche-culotte ou une protection contre l'incontinence, comprenant une feuille antérieure (3) perméable aux liquides, une feuille postérieure (2) imperméable aux liquides et un corps absorbant (4) enfermé entre elles, l'article présentant une partie avant (5), une partie arrière (6) et une partie entrejambe (7) agencée entre elles, et présentant en outre des parties de ceinture (9), attachées à la partie arrière (6) alternativement à la partie avant (5) de l'article et qui sont destinées, grâce à des premiers moyens de fixation (10), à être attachées autour de la taille de l'utilisateur et où ladite partie avant (5) alternativement la partie arrière (6) présente des deuxièmes moyens de fixation (8) destinés à être attachés contre les parties de ceinture (9) de telle manière que l'article prend une forme de type culotte dans laquelle les parties de ceinture (9) forment une partie des parties de taille de la culotte,
**caractérisé en ce que** lesdites parties de ceinture (9) comprennent une pluralité d'éléments de ceinture (11) dont l'étendue principale se trouve dans une direction transversale (y) de la ceinture et qui sont mutuellement connectés au moyen de fils ou de bandes (12), disposé(e)s selon la direction longitudinale (x) de la ceinture.

2. Article absorbant selon la revendication 1, **caractérisé en ce que** les fils/bandes (12) s'étendent de manière discontinue le long des parties de ceinture (9).

3. Article absorbant selon la revendication 1 ou 2, **caractérisé en ce que** lesdits fils ou bandes (12) sont élastiques.

4. Article absorbant selon la revendication 1 ou 2, **caractérisé en ce que** lesdits fils ou bandes (12) ne sont pas élastiques.

5. Article absorbant selon la revendication 1 ou 2, **caractérisé en ce que** lesdits fils ou bandes (12) comprennent partiellement des fils/bandes non élastiques et comprennent partiellement des fils/bandes élastiques.

6. Article absorbant selon l'une quelconque des revendications précédentes, **caractérisé en ce qu'**une partie de bord supérieur (17) des parties de ceinture (9) et/ou une partie de bord inférieur (18) des parties de ceinture (9) présentent entièrement ou partiellement un passepoil (16).

7. Article absorbant selon l'une quelconque des revendications précédentes, **caractérisé en ce que** les éléments de ceinture (11) font saillie dans la direction transversale (y) de la ceinture depuis des fils/bandes extérieur(e)s (12) des parties de ceinture (9).

8. Article absorbant selon l'une quelconque des revendications précédentes, **caractérisé en ce que** les éléments de ceinture (11) présentent différentes longueurs (13) dans la direction transversale (y) de la ceinture suivant la direction longitudinale (x) de la ceinture.

9. Article absorbant selon l'une quelconque des revendications précédentes, **caractérisé en ce que** les éléments de ceinture (11) présentent différentes largeurs (19) suivant la direction longitudinale (x) de la ceinture.

10. Article absorbant selon l'une quelconque des revendications précédentes, **caractérisé en ce que** lesdits fils/bandes (12) sont attaché(e)s sur l'extérieur des éléments de ceinture (11) alternativement à travers lesdits éléments de ceinture (11), de sorte que lesdits fils/bandes (12) ne se trouvent pas près de la peau de l'utilisateur lors de l'utilisation de ladite ceinture.
